# EUROPEAN PATENT APPLICATION

(11) **EP 4 005 558 A1**
(43) Date of publication of application: **01.06.2022**
(21) Application number: 20846340.6
(22) Date of filing: 31.07.2020
(51) Int. Cl.: A61K 9/14, A61K 9/16, A61K 47/22, A61K 47/69, A61K 9/00, A61K 38/00, A61P 35/00

(54) **ANTICANCER AGENT AND METHOD FOR PREPARATION OF POROUS SILICA PARTICLE**

(30) Priority: 31.07.2019 US 201962880733 P
(71) Applicant: Lemonex Inc., Seoul 08826 (KR)
(72) Inventor: WON, Cheol Hee, Seoul 08741 (KR)
(74) Representative: dompatent von Kreisler Selting Werner - Partnerschaft von Patent- und Rechtsanwälten mbB
(86) International application number: PCT/KR2020/010168
(87) International publication number: WO 2021/020945

(57) **Abstract**

The present invention provides an anticancer drug comprising a plurality of porous silica particles having an anticancer active peptide incorporated therein, wherein a plurality of nitrogen-containing groups are located on an outer surface of each porous silica particle, and folic acid is bound to at least some of the nitrogen-containing groups, whereby the anticancer drug has an excellent target delivery ability, is of high stability without forming a precipitate in an *in-vivo* environment, and is applicable to various carcinomas.

## Description

### [Technical Field]

The present invention relates to an anticancer drug and a method for preparation of porous silica particles.

### [Background Art]

Breast cancer is one of the most common cancers in the world, and it is known that an expression level of hormone receptors such as ER, PR, HER2, etc. on the surface of cancer cells is very important for diagnosis and treatment. Therefore, one of the traditional treatments is to use antibodies targeting these receptors or proteins. However, effectiveness of these treatments varies depending on biomarkers on the cell surface. As an alternative to these treatments, autophagy has been an attractive attempt.

Autophagy is a mechanism that decomposes proteins or organelles in cells to maintain homeostasis or supplement nutrients in the cells. Since autophagy is an essential mechanism to keep cells healthy, imbalanced autophagy may cause cell malfunction. Some cancers such as breast cancer are known as involving autophagy deficiency. In this case, autophagy is closely related to tumor formation. Beclin1 protein, which plays an important role in initiation of autophagy bodies ("autophagosome") during PI3KC3 composite formation, has received attention for its application in breast cancer treatment. In particular, a Bec1 peptide having 18 amino acids was identified, which exhibited similar activity and therapeutic efficacy to the Beclin1 protein. Therefore, the autophagy-inducing peptide, and Becl became a new therapeutic candidate for breast cancer.

However, hydrophobic nature of the peptide has been a major obstacle to the delivery of the becl peptide to breast cancer cells. To overcome this problem, cell permeable peptides such as TAT9 and 10 were used together. However, these methods were unable to achieve targeted delivery of bec1 into cancer cells.

### [Prior Art Document]

### [Patent Document]

Korean Patent Laid-Open Publication No. 2018-0130364

### [Summary of Invention]

### [Problems to be Solved by Invention]

An object of the present invention is to provide an anticancer drug.

Another object of the present invention is to provide a method for preparation of porous silica particles.

### [Means for Solving Problems]

To achieve the above objects, the following technical solutions are adopted in the present invention.
1. An anticancer drug comprising a plurality of porous silica particles having an anticancer active peptide incorporated therein, wherein a plurality of nitrogen-containing groups are located on an outer surface of each porous silica particle, and folic acid is bound to at least some of the nitrogen-containing groups.
2. The anticancer drug according to the above 1, wherein the folic acid is bound to 0.9% or less of the nitrogen-containing groups.
3. The anticancer drug according to the above 1, wherein the folic acid is bound to 11% or more of the nitrogen-containing groups.
4. The anticancer drug according to the above 1, wherein the folic acid is bound to 0.001% to 0.3% of the nitrogen-containing groups.
5. The anticancer drug according to the above 1, wherein the anticancer active peptide is bound to the porous silica particles through a disulfide bond.
6. The anticancer drug according to the above 1, wherein the porous silica particles include a plurality of irregularly arranged pores.
7. The anticancer drug according to the above 1, wherein the porous silica particles have a particle diameter of 50 to 500 nm and a pore diameter of 7 to 25 nm.
8. The anticancer drug according to the above 1, wherein the anticancer active peptide has a length of 5aa to 50aa in length.
9. The anticancer drug according to the above 1, wherein the anticancer active peptide includes a linker having an amino acid sequence of C(GG)n (n is 1 to 3) at a terminal thereof.
10. The anticancer drug according to the above 1, wherein the anticancer active peptide has an amino acid sequence of SEQ ID NO: 1.
11. The anticancer drug according to the above 1, wherein the porous silica particles have a BET surface area of 280 m²/g to 680 m²/g.
12. The anticancer drug according to the above 1, wherein the anticancer active peptide is incorporated in the porous silica particles in a weight ratio of 1:1 to 20.
13. The anticancer drug according to the above 1, wherein the anticancer drug is an injectable formulation.
14. The anticancer drug according to the above 1, wherein the cancer is breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, bone cancer, hair cell cancer, oral cancer, lip cancer, tongue cancer, salivary gland cancer, pharyngeal cancer, small intestine cancer, colon cancer, rectal cancer, kidney cancer, prostate cancer, vulvar cancer, thyroid cancer, bowel cancer, endometrial cancer, uterine cancer, brain cancer, central nervous system cancer, peritoneal cancer, hepatocellular carcinoma, Hodgkin disease or leukemia.
15. The anticancer drug according to the above 1, wherein an amount of nitrogen atoms on the outer surface of the porous silica particles is 0.1 mmol/g or more.
16. A method for preparation of porous silica particles comprising an anticancer active peptide incorporated therein, the method comprising: a first step of introducing nitrogen-containing groups on an outer surface of the porous silica particles in a state in which inner pores of the porous silica particles are filled with a surfactant; a second step of binding folic acid to at least some of the nitrogen-containing groups; a third step of removing the surfactant filled in the inner pores of the porous silica particles; and a fourth step of incorporating active peptide in the porous silica particles.
17. The method according to the above 16, wherein the surfactant is selected from the group consisting of cetyltrimethylammonium bromide (CTAB), hexadecyltrimethylammonium bromide (TMABr), hexadecyltrimethylpyridinium chloride (TMPrCl), and tetramethylammonium chloride (TMACl).
18. The method according to the above 16, wherein the active peptide is bound to the porous silica particles in a weight ratio of 1:1 to 20.
19. The method according to the above 16, wherein the porous silica particles are treated with the folic acid in an amount of 0.01 to 10 parts by weight based on 100 parts by weight of the porous silica particles.
20. The method according to the above 16, further comprising a step of preparing small pore silica particles in which small pores are filled with the surfactant by adding the surfactant and a silica precursor in a solvent and then stirring the same prior to the first step.
21. The method according to the above 20, further comprising a step of expanding the small pores by reacting the small pore silica particles with a swelling agent prior to the first step.
22. A method for production of the anticancer drug according to any one of the above 1 to 15, comprising the method for preparation of porous silica particles according to any one of the above 16 to 21.

### [Advantageous Effects]

The anticancer drug of the present invention is excellent in terms of target delivery ability of the anticancer active peptide to cancer.

The anticancer drug of the present invention does not form a precipitate in an *in-vivo* environment and has excellent stability.

The anticancer drug of the present invention may be applicable to various carcinomas.

### [Brief Description of Drawings]

FIG. 1 is a schematic diagram illustrating a FabBALL carrying a Becl peptide and the delivery of a protein to a target cell using the same.
FIG. 2 is a micrograph of porous silica particles according to an embodiment of the present invention.
FIG. 3 is a micrograph of porous silica particles according to another embodiment of the present invention.
FIG. 4 is a micrograph of small pore particles in a manufacturing process of porous silica particles according to an embodiment of the present invention.
FIG. 5 is a micrograph of small pore particles according to an embodiment of the present invention.
FIG. 6 is each micrograph of porous silica particles relative to pore diameters according to an embodiment of the present invention. A degradable delivery vehicle (DDV) is the particle according to the embodiment, wherein the number in parenthesis indicates a diameter of the particle and the number of subscripts indicates the pore diameter. For example, DDV(200)₁₀ refers to a particle having a particle diameter (that is, particle size) of 200 nm and a pore diameter of 10 nm according to an embodiment.
FIG. 7 illustrates features of BALL and peptide, wherein (a) is a TEM image of FabBALL (scale bar = 200 nm), (b) is a hydrodynamic particle size distribution of FabBALL, (c) is a zeta potential of each surface-modified BALL, and (d) is a mass spectrum of Becl peptide measured by MALDI-TOF MS.
FIG. 8 illustrates characteristics of FabBALL, wherein (a) is a loading efficiency measured by UV-vis absorption of the released 4-thiopyridone (324 nm), (b) is an emission profile of fluorescein-conjugated Becl peptide measured by fluorescence (ex/em: 492/517), (c) illustrates localization in cells treated with FabBALL carrying Becl peptide for 12 hours (blue: DAPI, green: fluorescein-labeled Becl, red: Cy3-labeled FabBALL, scale bar =15 µm).
FIG. 9 demonstrates that FabBALL causes autophagy-mediated cell death, wherein (a) is a survival rate of MCF-7 cells when various concentrations of Becl peptide were used for treatment alone or with FabBALL, (b) is a survival rate of MCF-7 cells treated with various concentrations of FabBALL, abBALL+bec1 and FabBALL+bec1, respectively, (c) is a fluorescence image of MCF-7 cells expressing LC3-GFP treated with PBS, becl, FabBALL and FabBALL+bec1, respectively (blue: DAPI, green: GFP, scale bar = 20 µm), and (d) is the number of LC3 puncta in each group (n = 100).
FIGS. 10 to 12 illustrate anticancer activity against various cancer cell lines, wherein (a) is the prostate cancer cell line PC-3, (b) is the prostate cancer cell line LNCaP, and (c) illustrates results of the ovarian cancer cell line HeLa.
FIG. 13 illustrates results of observation of solution stability according to a ratio of folic acid to nitrogen-containing groups in particles.
FIG. 14 is a schematic diagram illustrating arrangement of pores of the porous silica particles.
FIG. 15 is a TEM image of the porous silica particles.

### [Mode for Carrying out Invention]

Hereinafter, the present invention will be described in detail.

The present invention relates to an anticancer drug.

The anticancer drug of the present invention may include a plurality of porous silica particles in which anticancer active peptides are incorporated, wherein a plurality of nitrogen-containing groups are located on the outer surface of each porous silica particle, and folic acid is bound to at least some of the nitrogen-containing groups.

The porous silica particles according to the present invention may include anticancer active peptide incorporated therein. The anticancer active peptide may be loaded inside the pores.

For example, the anticancer active peptide may be bound inside the pores by a disulfide bond. In cancer cells, glutathione is present at a high concentration in lysosome of the cell. Therefore, the disulfide bond may be broken in the cancer cell and the anticancer active peptide may be specifically released.

The disulfide bond may be formed, for example, by a reaction of a sulfur-containing group in the pores of the porous silica particles with a sulfur-containing group at the terminal of the anticancer active peptide. The sulfur-containing group may be, for example, a mercapto group, a mercaptoalkyl group, etc.

Porous silica particles may have a high loading rate of anticancer active peptides due to a high ratio of sulfur-containing groups in the pores. For example, the ratio of sulfur atoms in the pores may be 0.05 mmol/g or more, and specifically, 0.1 mmol/g or more, 0.2 mmol/g or more, 0.3 mmol/g or more, etc., but it is not limited thereto. The upper limit may be, for example, 1 mmol/g, 0.7 mmol/g, 0.5 mmol/g, 0.4 mmol/g, etc. This may be a value determined through elemental analysis.

The anticancer active peptide may include a C(GG)n (n is 1 to 3) linker at the terminal.

Cysteine (C) of the linker may be used to form a disulfide bond, and diglycine (GG) enables the peptide to be separated from the inside of the pores at a proper distance without affecting the function thereof, such that the peptide can be more easily released from the porous silica particles.

Type of the anticancer active peptide is not limited as long as it has anticancer activity and a functional group capable of forming a disulfide bond or can bind such a functional group. For example, the above peptide may be a peptide consisting of the sequence of SEQ ID NO: 1. With respect to the disulfide bond, for example, the anticancer active peptide may have a cysteine (C) at the N-terminal or C-terminal.

As the anticancer active peptide, any one known to have anticancer activity for carcinoma to which the peptide is applied may be used without limitation thereof. Further, if the known peptide is unable to involve disulfide bonds, for example, a peptide including cysteine additionally bound at the N-terminal or C-terminal of the corresponding sequence may be used.

The anticancer active peptide may have a length of, for example, 5aa to 50aa, and specifically, 5aa to 40aa, 5aa to 30aa, 8aa to 25aa, 10aa to 25aa, 12aa to 25aa, 15aa to 25aa, etc., but it is not limited thereto. The length may be a length including a linker.

The anticancer active peptide may further have a functional group known in the art for improvement of pharmacokinetics (PKs) at the N-terminal or C-terminal. This may be, for example, fatty acid, cholesterol, alkyl group, polyethylene glycol, etc., but it is not limited thereto. The fatty acid may be, for example, a fatty acid having 8 to 22 carbon atoms, while the alkyl group may be, for example, an alkyl group having 1 to 20 carbon atoms.

The anticancer active peptide may be a composite of two or more peptides. For example, a plurality of peptides may be bound with a linker known in the art such as a disulfide bond, but it is not limited thereto.

A loading ratio of the anticancer active peptide in the particles, for example, a weight ratio of anticancer active peptide to porous silica particles may be 1:1 to 20. When the weight ratio is within the above range, the peptide is sufficiently loaded, while generation of empty porous silica particles not carrying peptide may be prevented. Within the above range, for example, 1:1 to 20, 1:3 to 20, 1:3 to 15, 1:5 to 15, 1:6 to 14, 1:6 to 12, 1:6 to 10, etc., but it is not limited thereto.

Porous silica particles may be introduced into cancer cells by binding folic acid to the outer surface of the particles, which in turn specifically bind to folate receptors on the surface of cancer cells.

Porous silica nanoparticles may have nitrogen-containing groups on the outer surface thereof, thereby enabling folic acid to be bound to the outer surface.

The porous silica nanoparticles may have a high proportion of nitrogen-containing groups on the outer surface thereof. For example, a ratio of nitrogen atoms on the outer surface may be 0.1 mmol/g or more, and specifically, 0.5 mmol/g or more, 1 mmol/g or more, 1.5 mmol/g or more, 2 mmol/g or more, etc., but it is not limited thereto. The upper limit may be, for example, 10 mmol/g, 5 mmol/g, 3 mmol/g, 2.5 mmol/g, etc. This may be a value determined through elemental analysis.

The porous silica particles may be surface-modified by treating the particles with a compound for introducing the substituent so as to have nitrogen-containing groups as much as possible on the outer surface.

The nitrogen-containing group may be, for example, an amino group or aminoalkyl group. For example, this may be produced by binding an aminoalkyl group to a silanol group on the outer surface of the porous silica particles. The aminoalkyl group may be, for example, an aminopropyl group. The nitrogen-containing group may be formed, for example, by treating the outer surface of the porous silica particles with alkoxysilane having the nitrogen-containing group. Specifically, the nitrogen-containing group may be N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silan, 3-(2-aminoethylamino)propyldimethoxymethylsilane and the like.

The compound having a nitrogen-containing group may be used for treating the porous silica particles in an amount of, for example, 0.1 to 10 parts by weight ("wt. parts"), specifically 0.1 to 5 wt. parts, more specifically 1 to 5 wt. parts, and most specifically 1 to 3 wt. parts based on 100 wt. parts of the particles, but it is not limited thereto.

Folic acid on the outer surface of the porous silica particles is bound to at least some of the nitrogen-containing groups present on the outer surface. Thereby, the porous silica particles do not form a precipitate and may exhibit excellent dispersion stability. For example, folic acid may be bound to 0.9% or less or 11% or more of the nitrogen-containing groups. Specifically, folic acid may be bound to 0.9% or less, 0.8% or less, 0.7% or less, 0.6% or less, 0.5% or less, 0.4% or less, 0.3% or less, 0.2% or less, or 0.1% or less of the nitrogen-containing groups. In such a case, the lower limit may be, for example, 0.001%, 0.005%, 0.01%, 0.03%, 0.05%, etc., but it is not limited thereto. Folic acid may be combined to satisfy all possible combinations of the upper and lower limits. In addition, specifically, folic acid may be bound to 11% or more, 15% or more, 20% or more, 30% or more, 40% or more, 50% or more, 60% or more, 70% or more, 80% or more, 90% or more of the nitrogen-containing groups. The upper limit may be 100%, but it is not limited thereto.

The binding ratio can be adjusted, for example, by changing the amount of folic acid used for treatment.

The binding ratio may be calculated as an added amount of folic acid during reaction relative to the amount of nitrogen-containing groups introduced to the outer surface of the porous silica particles, for example, the amount of nitrogen-containing groups may be an amount derived through elemental analysis. To take a specific example, when the nitrogen atom ratio of the outer surface of the porous silica particles is 2.1 mmol/g as a result of elemental analysis, in order to adjust the folic acid ratio to 0.1% (molar ratio) of the nitrogen-containing groups, 0.046 mg of folic acid can be added to 50 mg of the particles for reaction, but it is not limited thereto.

The combination of folic acid and the nitrogen-containing group may be formed by, for example, an amide bond, and specifically, may be performed by EDC coupling, but it is not limited thereto.

The porous silica particles may be made of a silica (SiO₂) material, and may have a nano-sized particle diameter.

The porous silica particles are porous particles, have nano-sized pores, and may load anticancer active peptides on the surface and/or inside the pores.

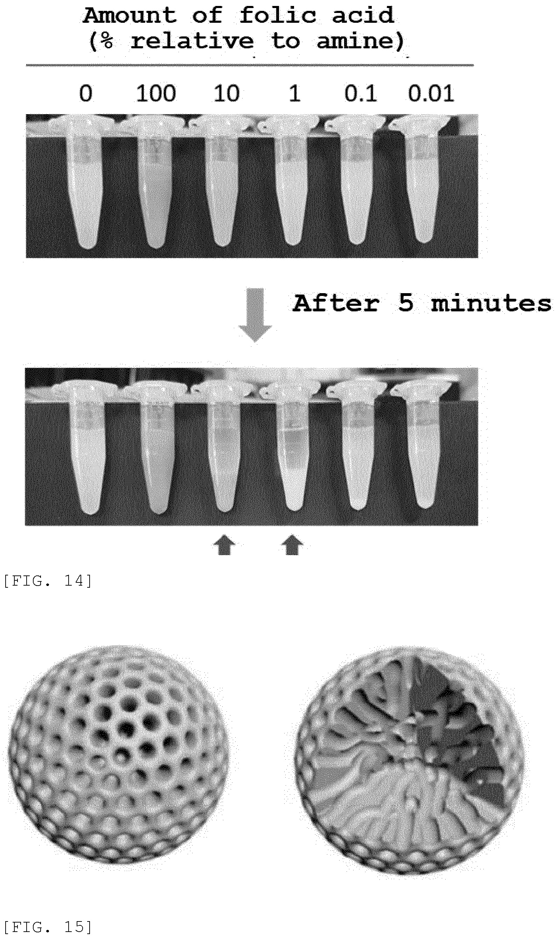

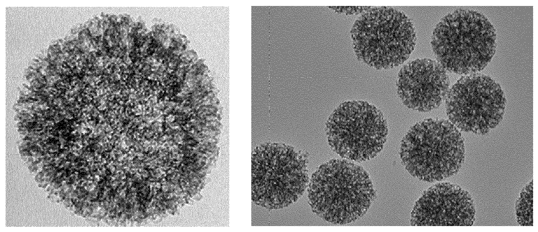

The pores of the porous silica particles may be irregularly arranged. Commonly, the porous silica particles have an ordered pore structure, whereas the porous silica particles according to the present invention may have a non-ordered pore structure. Thereby, a path through which the anticancer active peptide is released becomes more irregular and longer, such that the peptide may be released more sustainedly. The porous silica particles may have irregular pore arrangements, for example, as illustrated in FIGS. 14 and 15, but they are not limited thereto.

The porous silica particles may have an average pore diameter of 7 to 25 nm. The average pore diameter may be, for example, 7 to 25 nm within the above range. Within the above range, the average pore diameter may be, for example, 7 to 25 nm, 7 to 23 nm, 10 to 25 nm, 13 to 25 nm, 7 to 20 nm, 7 to 18 nm, 10 to 20 nm, 10 to 18 nm, etc., but it is not limited thereto.

The porous silica particles may be, for example, spherical particles, but they are not limited thereto.

The porous silica particles may have, for example, a particle diameter of 50 to 500 nm. Within the above range, the particle diameter may be, for example, 50 to 500 nm, 50 to 400 nm, 50 to 300 nm, 100 to 450 nm, 100 to 400 nm, 100 to 350 nm, 100 to 300 nm, 150 to 400 nm, 150 to 350 nm, 200 to 400 nm, 200 to 350 nm, 250 to 400 nm, 180 to 300 nm, 150 to 250 nm, etc., but it is not limited thereto.

The porous silica particles may have, for example, a BET surface area of 280 to 680 m²/g. For example, within the above range, the BET surface area may be 280 to 680 m²/g, 280 to 600 m²/g, 280 to 500 m²/g, 280 to 400 m²/g, 300 to 650 m²/g, 300 to 600 m²/g, 300 to 550 m²/g, 300 to 500 m²/g, 300 to 450 m²/g, 350 to 450 m²/g, etc., but it is not limited thereto.

The pores of the porous silica particles may have a volume per gram of, for example, 0.7 to 2.2 ml. For example, within the above range, the volume per gram of the pore may be 0.7 to 2.0 ml, 0.8 to 2.2 ml, 0,8 to 2.0 ml, 0.9 to 2.0 ml, 1.0 to 2.0 ml, etc., but it is not limited thereto. If the volume per gram is excessively small, a degradation rate may be too high, and it may be difficult to produce excessively large particles, or the particles may not have an intact shape.

With regard to the porous silica particles, small pore particles may include expanded pores with an average pore diameter of 7 to 25 nm. As a result, a large or long peptide can be loaded inside the pores having a large pore diameter. Further, since a particle size itself is not large compared to the pore diameter, whereby it is easy to deliver and absorb the peptide into cells.

The combination of the anticancer active peptide and folic acid may include: modifying an outer surface of the porous silica particles filled with a surfactant inside the pores of the particle, in order to have a nitrogen-containing group on the outer surface; binding folic acid to the nitrogen-containing group; removing the surfactant contained in the pores of the porous silica particles; and combining the anticancer active peptide in the pores by a disulfide bond.

The porous silica particles may be prepared, for example, through processes of forming small pores and pore expansion. In this case, the small pore particles may be obtained by stirring and homogenizing a surfactant and a silica precursor in a solvent. The pore expansion may be performed by treating the obtained small pore particles with a pore swelling agent.

In this case, particles are obtained with a surfactant filled inside the pores. At this time, the outer surface of the pores may be modified to have a nitrogen-containing group. This may be performed by treating the particles with a compound having a nitrogen-containing group, as described above. Surface modification is performed in a state in which the surfactant is filled in the pores, whereby it may be modified to have nitrogen-containing groups only outside the pores rather than inside the pores.

Thereafter, the porous silica particles may be treated with folic acid to bind folic acid to the nitrogen-containing group.

For example, folic acid treatment may satisfy the above-described ratio.

Thereafter, the surfactant inside the pores is removed in order to bind the anticancer active peptide inside the pores.

Removal of the surfactant inside the pores may be performed by acid treatment, and specifically, may be performed by treating with acid-containing alcohol. The acid may be, for example, hydrochloric acid, but it is not limited thereto.

The alcohol may be, for example, an alcohol having 1 to 3 carbon atoms, and specifically, ethanol.

The acid treatment may be performed under stirring, for example, for 4 to 24 hours, specifically 8 to 24 hours, and more specifically 12 to 20 hours.

The acid treatment may be performed under heating, for example, at 80 to 150 °C, specifically 90 to 130 °C.

Thereafter, the anticancer active peptide may be bound to the inside of the pore through a disulfide bond.

In order to form a disulfide bond, the porous silica particles may be modified to have a sulfur-containing group inside the pores.

This may be performed, for example, by treating a compound having a sulfur-containing group.

The compound may be an alkoxysilane having a sulfur-containing group, and specifically (3-mercaptopropyl) trimethoxysilane, but it is not limited thereto.

When the particles are treated with the anticancer active peptide having a sulfur-containing group, a disulfide bond is formed and thus the anticancer active peptide may be combined

The combination of an anticancer active peptide may be performed by mixing porous silica particles in a solvent with anticancer active peptides.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like.

Further, phosphate-buffered saline (PBS) solution, simulated body fluid (SBF), borate-buffered saline, tris-buffered saline may be used as the solvent.

Surface modification may be performed by reacting the porous silica particles, which were dispersed in a solvent, with the compound described above.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The reaction of the porous silica particles with the above-described compound may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous silica particles with the above-described compound may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

Washing may be carried out between respective processes.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

Specific examples of a method for preparing particles will be described below.

The small pore particles may be, for example, particles having an average pore diameter of 1 to 5 nm.

The small pore particles may be obtained by adding a surfactant and a silica precursor in a solvent, followed by agitation.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and the organic solvent, a relative ratio of water and organic solvent may be, for example, in a volume ratio of 1:0.7 to 1.5, for example, 1:0.8 to 1.3, but it is not limited thereto.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

The surfactant may be added, for example, in an amount of 1 to 10 g, for example, 1 to 8 g, 2 to 8 g or 3 to 8 g per liter of solvent within the above range, but it is not limited thereto.

The silica precursor may be added after stirring with addition of a surfactant to the solvent. The silica precursor may be, for example, tetramethyl orthosilicate (TMOS), tetraethyl orthosilicate (TEOS), and the like, but it is not limited thereto.

The stirring may be conducted, for example, for 10 to 30 minutes, but it is not limited thereto

The silica precursor may be added in an amount of 0.5 to 5 ml per liter of solvent, for example, 0.5 ml to 4 ml, 0.5 to 3 ml, 0.5 to 2 ml, 1 to 2 ml, etc. within the above range, but it is not limited thereto.

If necessary, sodium hydroxide may further be used as a catalyst, and specifically, may be added under stirring after addition of the surfactant and before addition of the silica precursor to the solvent.

The sodium hydroxide may be added in an amount of 0.5 to 8 ml per liter of solvent, for example, 0.5 to 5 ml, 0.5 to 4 ml, 1 to 4 ml, 1 to 3 ml, 2 to 3 ml, etc. within the above range based on 1 M aqueous sodium hydroxide solution, but it is not limited thereto.

After addition of the silica precursor, the solution may be reacted with stirring. The stirring may be conducted for 2 to 15 hours, for example, 3 to 15 hours, 4 to 15 hours, 4 to 13 hours, 5 to 12 hours, 6 to 12 hours, 6 to 10 hours, etc. within the above range, but it is not limited thereto. If the stirring time (reaction time) is too short, nucleation may be insufficient.

After agitation, the solution may be aged. Aging may be performed for 8 to 24 hours, for example, for 8 to 20 hours, 8 to 18 hours, 8 to 16 hours, 8 to 14 hours, 10 to 16 hours, 10 to 14 hours, etc. within the above range, but it is not limited thereto.

Thereafter, the reaction product may be washed and dried to harvest porous silica particles and, if necessary, separation of unreacted material may proceed before washing.

Separation of the unreacted material may be implemented by separating the supernatant, for example, through centrifugation. For example, centrifugation may be conducted at 6,000 to 10,000 rpm, and the centrifugation time may range from 3 to 60 minutes, for example, 3 to 30 minutes, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

In the particles produced by the above-described method, residual organic substances (surfactants, etc.) used for the reaction may remain on the surface and inside the pores, and washing may be performed to remove the same. Commonly, acid treatment (or acidic organic solvent treatment) may be performed to remove such organic substances, but in the present invention, since such acid treatment is not performed, residual organic substances may remain in the pores even after washing.

The drying may be conducted, for example, at 20 to 100 °C, but it is not limited thereto, and may also be conducted in a vacuum state.

Thereafter, the pore of the harvested porous silica particles may be expanded, and such pore expansion may be conducted using a pore swelling agent.

The pore swelling agent may include, for example, trimethylbenzene, triethylbenzene, tripropylbenzene, tributylbenzene, tripentylbenzene, trihexylbenzene, toluene, benzene, etc., and specifically, trimethylbenzene may be used, but it is not limited thereto.

Further, the pore swelling agent used herein may be, for example, N, N-dimethylhexadecylamine (DMHA), but it is not limited thereto.

The pore expansion may be performed, for example, by mixing the porous silica particles in the solvent with a pore swelling agent and heating the mixture to induce reaction.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The porous silica particles may be added in a ratio of 10 to 200 g per liter of solvent, for example, 10 to 150 g, 10 to 100 g, 30 to 100 g, 40 to 100 g, 50 to 100 g, 50 to 80 g, 60 to 80 g, etc., within the above range, but it is not limited thereto.

The porous silica particles may be evenly dispersed in a solvent. For example, the porous silica particles may be added to the solvent and ultrasonically dispersed. In the case of using a mixed solvent, the porous silica particles may be dispersed in a first solvent, followed by adding a second solvent thereto.

The pore swelling agent may be added in a ratio of 10 to 200 parts by volume ("vol. parts") based on 100 vol. parts of solvent, for example, 10 to 150 vol. parts, 10 to 100 vol. parts, 10 to 80 vol. parts, 30 to 80 vol. parts, 30 to 70 vol. parts, etc. within the above range, but it is not limited thereto.

The reaction may be carried out at 120 to 190 °C, for example, 120 to 190 °C, 120 to 180 °C, 120 to 170 °C, 130 to 170 °C, 130 to 160 °C, 130 to 150 °C, 130 to 140 °C, etc. within the above range, but it is not limited thereto.

The reaction may be carried out for 6 to 96 hours, for example, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, 6 to 96 hours, 7 to 96 hours, 8 to 80 hours, 9 to 72 hours, 9 to 80 hours, 6 to 72 hours, 9 to 96 hours, 10 to 80 hours, 10 to 72 hours, 12 to 66 hours, 13 to 60 hours, 14 to 96 hours, 15 to 88 hours, 16 to 80 hours, 17 to 72 hours, etc. within the above range, but it is not limited thereto.

The time and temperature may be desirably adjusted within the ranges exemplified above so that the reaction may be carried out sufficiently but not excessively. For example, when the reaction temperature is reduced, the reaction time may be increased, and when the reaction temperature is increased, the reaction time may be shortened. If the reaction is not sufficiently performed, pore expansion may be insufficient. On the other hand, if the reaction proceeds excessively, the particles may collapse due to overexpansion of the pores.

The reaction may be carried out, for example, by gradually raising the temperature. Specifically, the reaction may be carried out by gradually raising the temperature at a rate of 0.5 to 15 °C/min from the room temperature to the above-defined temperature. For example, the temperature may be raised at a rate of 1 to 15 °C/min, 3 to 15 °C/min, 3 to 12 °C/min, 3 to 10 °C/min, etc., but it is not limited thereto.

The reaction may be carried out under stirring. For example, the stirring may be implemented at a speed of 100 rpm or more, and specifically, at a speed of 100 to 1000 rpm, but it is not limited thereto.

After the reaction, the reaction solution may be cooled slowly, for example, by gradually decreasing the temperature. Specifically, the reaction may be carried out by gradually decreasing the temperature at a rate of 0.5 to 20 °C/min from the above-defined temperature to room temperature. For example, the temperature may be decreased at a rate of 1 to 20 °C/min, 3 to 20 °C/min, 3 to 12 °C/min, 3 to 10 °C/min, etc. within the above range, but it is not limited thereto.

After cooling, the reaction product may be washed and dried to harvest porous silica particles having expanded pores. If necessary, unreacted material may be first separated before washing.

Separation of the unreacted material may be implemented by separating the supernatant, for example, through centrifugation. Herein, centrifugation may be conducted, for example, at 6,000 to 10,000 rpm, and the centrifugation time may range from 3 minutes to 60 minutes. For example, the centrifugation may be conducted for 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times, 4 times, 5 times, 6 times, 7 times, 8 times, etc.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The anticancer drug of the present invention may stably deliver anticancer active peptide loaded therein into the body, and can release the same to a target in cancer cells.

Cancer as a target for delivery of the anticancer drug according to the present invention may be any cancer that can over-express a folate receptor on the surface of the cancer, and may include, for example, breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, kidney cancer, bone cancer, bone marrow disorder, lymphatic disorder, hair cell cancer, oral and pharyngeal (oral) cancer, lip cancer, tongue cancer, oral cancer, salivary gland cancer, pharyngeal cancer, small intestine cancer, colon cancer, rectal cancer, kidney cancer, prostate cancer, vulvar cancer, thyroid cancer, bowel cancer, endometrial cancer, uterine cancer, brain cancer, central nervous system cancer, peritoneal cancer, hepatocellular carcinoma, head cancer, neck cancer, Hodgkin disease, leukemia, etc., but it is not limited thereto.

The cancer may be an anticancer drug-resistant cancer, but it is not limited thereto.

The anticancer drug of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The anticancer drug of the present invention is applicable in a form of any formulation containing the nucleic acid molecule of the present invention as an active ingredient, and may be prepared in oral or parenteral formulations. The pharmaceutical formulations of the present invention may include forms suitable for oral, rectal, nasal, topical (including the cheek and sublingual), subcutaneous, vaginal or parenteral (intramuscular, subcutaneous) administration. Alternatively, forms suitable for administration by inhalation or insufflations may also be included. More specifically, the anticancer drug of the present invention does not form a precipitate in *in-vivo* environments or blood, and is possibly administered even using a thin injection needle. Therefore, the injectable formulation is preferably used in particular.

The anticancer drug of the present invention is administered in a pharmaceutically effective amount. Effective dose levels may be determined depending on types of disease of the patient, severity, activity of drug, sensitivity to drug, administration time, administration route and rate of release, duration of treatment, factors including concurrent medications, and other factors well known in the medical field. The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the anticancer drug according to the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the carrier of the present invention used. For example, the amount may be from 0.01 µg to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The anticancer drug of the present invention may further include a compound to maintain/increase one or more of active ingredients exhibiting the same or similar functions in relation to treatment of wounds or the solubility and/or absorption of at least one active ingredient.

Further, the anticancer drug of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

Further, the present invention relates to a method for production of porous silica particles containing active peptide therein.

The method of the present invention may include: a first step of introducing nitrogen-containing groups on an outer surface of the porous silica particles in a state in which inner pores of the porous silica particles are filled with a surfactant; a second step of binding folic acid to at least some of the nitrogen-containing groups; a third step of removing the surfactant filled in the inner pores of the porous silica particles; and a fourth step of incorporating active peptide into the porous silica particles.

In the first step, the nitrogen-containing group may be introduced on the outer surface of the porous silica particles while filling the inner pores of the porous silica particles with a surfactant.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

By surface modification in a state in which the pores are filled with the surfactant, the particles may be modified to have the nitrogen-containing group only on an outer portion of the pores rather than the inside of the pores.

The nitrogen-containing group may include, for example, an amino group or aminoalkyl group. The nitrogen-containing group may be produced, for example, by combining an aminoalkyl group with a silanol group on the outer surface of the porous silica particles. The aminoalkyl group may be, for example, an aminopropyl group. The nitrogen-containing group may be obtained, for example, by treating the outer surface of the porous silica particles with alkoxysilane containing the nitrogen-containing group. Specifically, the nitrogen-containing group may include, for example, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silan, 3-(2-aminoethylamino)propyldimethoxymethylsilane and the like.

The compound having a nitrogen-containing group may be used for treating the porous silica particles in an amount of, for example, 0.1 to 10 wt. parts, specifically 0.1 to 5 wt. parts, more specifically 1 to 5 wt. parts, and most specifically 1 to 3 wt. parts based on 100 wt. parts of the particles, but it is not limited thereto.

The porous silica particles may be produced according to the above-illustrated method.

The method of the present invention may further include preparing the porous silica particles illustrated above.

The porous silica particles may have the specifications illustrated above, but they are not limited thereto.

In the second step, folic acid may be bound to at least some of the nitrogen-containing groups.

Reacting the porous silica particles, which have nitrogen-containing groups on the outer surface thereof, with folic acid may enable the folic acid to be bound to the nitrogen-containing group.

Folic acid may be bound to, for example, 0.9% or less or 11% or more of the nitrogen-containing groups. Specifically, folic acid may be bound to 0.9% or less, 0.8% or less, 0.7% or less, 0.6% or less, 0.5% or less, 0.4% or less, 0.3% or less, 0.2% or less, 0.1% or less of the nitrogen-containing groups.

In this case, the lower limit may be, for example, 0.001%, 0.005%, 0.01%, 0.03%, 0.05%, etc., but it is not limited thereto.

Folic acid can be combined to satisfy all possible combinations of the upper and lower limits. In addition, specifically, folic acid may be bound to 11% or more, 15% or more, 20% or more, 30% or more, 40% or more, 50% or more, 60% or more, 70% or more, 80% or more, 90% or more of the nitrogen-containing groups. The upper limit may be 100%, but it is not limited thereto.

A binding ratio may be adjusted, for example, by changing the amount of folic acid used for treatment.

The binding ratio may be calculated as an amount of folic acid added during the reaction relative to an the amount of nitrogen-containing groups introduced to the outer surface of the porous silica particles, for example, the amount of nitrogen-containing groups may be an amount derived through elemental analysis.

Folic acid may be used for treatment in an amount of, for example, 0.001 to 200 wt. parts, specifically 0.001 to 100 wt. parts, 0.001 to 50 wt. parts, 0.001 to 30 wt. parts, 0.001 to 10 wt. parts, 0.001 to 5 wt. parts, 0.001 to 3 wt. part, 0.001 to 1 wt. part, 0.001 to 0.5 wt. parts, 0.01 to 10 wt. parts, 0.01 to 5 wt. parts, 0.01 to 3 wt. parts, 0.01 to 2 wt. parts, 0.1 to 10 wt. parts, 0.1 to 5 wt. parts, 0.1 to 3 wt. parts, 0.1 to 2 wt. parts, 10 to 200 wt. parts, 10 to 150 wt. parts, 10 to 100 wt. parts, 20 to 200 wt. parts, 20 to 150 wt. parts, 20 to 100 wt. parts, 20 to 50 wt. parts, 30 to 200 wt. parts, 30 to 150 wt. parts, 30 to 100 wt. parts, 50 to 200 wt. parts, 50 to 150 wt. parts, 50 to 100 wt. parts, etc. based to 100 wt. parts of the porous silica particles, but it is not limited thereto.

In the third step, the surfactant filled in the pores of the porous silica particles is removed.

Removal of the surfactant in the pores may be performed, for example, by acid treatment, and specifically, may be performed by treating with acid-containing alcohol. The acid may be, for example, hydrochloric acid, but it is not limited thereto.

The alcohol may be, for example, an alcohol having 1 to 3 carbon atoms, and specifically, ethanol.

The acid treatment may be performed under stirring, for example, for 4 to 24 hours, specifically 8 to 24 hours, and more specifically 12 to 20 hours.

The acid treatment may be performed under heating at a temperature of, for example, 80 to 150 °C, and specifically 90 to 130 °C.

In the fourth step, the porous silica particles include active peptide incorporated therein.

The active peptide may be, for example, bound to the inside of pores through a disulfide bond.

In order to form a disulfide bond, the porous silica particles may be modified to have a sulfur-containing group inside the pores. This may be performed, for example, by treating the particles with a compound having a sulfur-containing group. The compound may be an alkoxysilane having a sulfur-containing group, and specifically (3-mercaptopropyl) trimethoxysilane, but it is not limited thereto.

When the active peptide having a sulfur-containing group is treated therein, a disulfide bond is formed thereby binding the anticancer active peptide

The binding of the active peptide may be performed, for example, by mixing the porous silica particles and the active peptide in a solvent.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like.

Further, phosphate-buffered saline (PBS) solution, simulated body fluid (SBF), borate-buffered saline, tris-buffered saline may be used as the solvent.

Surface modification may be performed by reacting, for example, the porous silica particles, which were dispersed in a solvent, with the above-described compound.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The reaction of the porous silica particles with the above-described compound may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous silica particles with the above-described compound may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

Washing may be carried out between respective processes.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

Hereinafter, the present invention will be described in detail with reference to the following examples.

### Example

### Example 1. Porous silica particles

### 1. Preparation of porous silica particles

### (1) Preparation of porous silica particles

### 1) Preparation of small pore particles

960 mL of distilled water (DW) and 810 mL of MeOH were put into a 2 L round bottom flask. 7.88 g of CTAB was added to the flask, followed by rapid addition of 4.52 mL of 1 M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8000 rpm and 25 °C for 10 minutes to remove the supernatant, centrifuged at 8000 rpm and 25 °C for 10 minutes, and washed five times with ethanol and distilled water alternately.

Thereafter, the resultant product was dried in an oven at 70 °C to harvest 1.5 g of powdery microporous silica particles (pore average diameter of 2 nm and particle size of 200 nm).

### 2) Pore expansion

1.5 g of microporous silica particle powder was added to 10 ml of ethanol and subjected to ultrasonic dispersion, and 10 ml of water and 10 ml of trimethyl benzene (TMB) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8000 rpm for 10 minutes at 25 °C to remove the supernatant, and centrifuged at 8000 rpm for 10 minutes at 25 °C and washed five times with ethanol and distilled water alternately.

Then, the product was dried in an oven at 70 °C to harvest powdery porous silica particles (pore diameter of 17.2 nm, and particle size of 200 nm).

### (2) Preparation of porous silica particles

Porous silica particles were prepared by the same method as Example 1-1-(1), except that the reaction conditions at the time of pore expansion were changed to 140 °C and 72 hours.

### (3) Preparation of porous silica particles (10 L scale)

Porous silica particles were prepared by the same method as Example 1-1-(1), except that a 5 times larger container was used and each material was used in a 5 times capacity.

### (4) Preparation of porous silica particles (particle size of 300 nm)

Porous silica particles were prepared by the same method as Example 1-1-(1), except that 920 ml of distilled water and 850 ml of methanol were used to prepare the small pore particles.

### (5) Preparation of porous silica particles (particle size of 500 nm)

Porous silica particles were prepared by the same method as Example 1-1-(1), except that 800 ml of distilled water, 1010 ml of methanol, and 10.6 g of CTAB were used to prepare the small pore particles.

### (6) Preparation of porous silica particles (particle size of 1000 nm)

Porous silica particles were prepared by the same method as Example 1-1-(1), except that 620 ml of distilled water, 1380 ml of methanol, and 7.88 g of CTAB were used to prepare the small pore particles.

### (7) Preparation of porous silica particles (pore diameter of 4 nm)

Porous silica particles were prepared by the same method as Example 1-1-(1), except that 2.5 mL of TMB was used for pore expansion.

### (8) Preparation of porous silica particles (pore diameter of 7 nm)

Porous silica particles were prepared by the same method as Example 1-1-(1), except that 4.5 mL of TMB was used for pore expansion.

### (9) Preparation of porous silica particles (pore diameter of 17 nm)

Porous silica particles were prepared by the same method as Example 1-1-(1), except that 11 mL of TMB was used for pore expansion.

### (10) Preparation of porous silica particles (pore diameter of 23 nm)

Porous silica particles were prepared by the same method as Example 1-1-(1), except that 12.5 mL of TMB was used for pore expansion.

### (11) Preparation of porous silica particles

Porous silica particles were prepared by the same method as Example 1-1-(1), except that 900 ml of distilled water, 850 mL of methanol and 8 g of CTAB were used in preparation of small pore particles.

### 2. Identification of particle formation and pore expansion

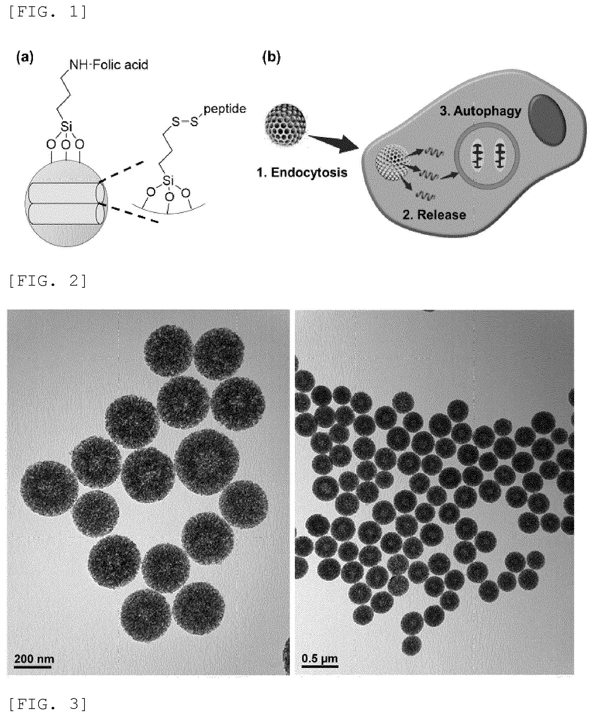

Small pore particles and porous silica particles prepared in Examples 1-1-(1) to (3) were observed under a microscope to determine whether the small pore particles were uniformly formed or the pores were sufficiently expanded to uniformly form the porous silica particles (FIGS. 2 to 5) .

FIG. 2 is photographs of the porous silica particles in Example 1-1-(1), and FIG. 3 is photographs of the porous silica particles in Example 1-1-(2), and from these drawings, it can be seen that spherical porous silica particles having sufficiently expanded pores were formed evenly.

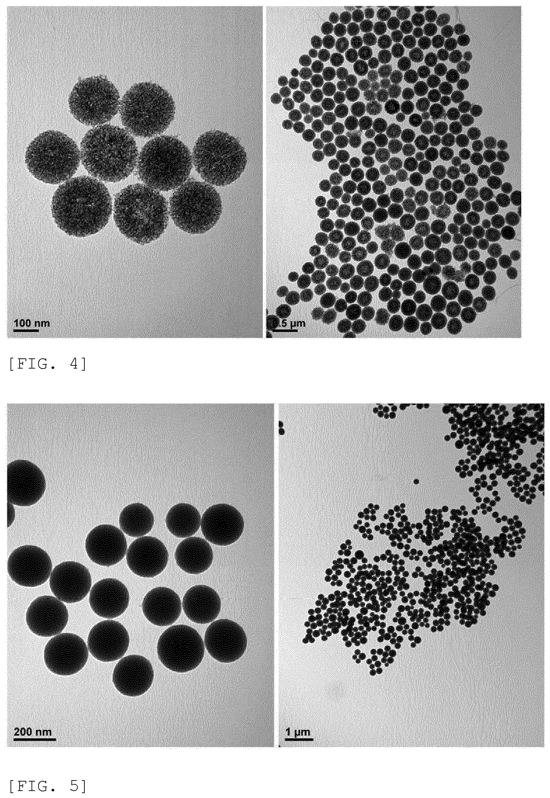

FIG. 4 is photographs of the small pore particles in Example 1-1-(1), and FIG. 5 is comparative photographs of the small pore particles in Examples 1-1-(1) and 1-1-(3), and from these drawings, it can be seen that spherical small pore particles were formed evenly.

### 3. Calculation of BET surface area and pore volume

Surface areas and pore volumes of the small pore particles in Example 1-1-(1) and the porous silica particles of Examples 1-1-(1), (7), (8), (10) and (11) were calculated. The surface areas were calculated by Brunauer-Emmett-Teller (BET) method, and the pore size distributions were calculated by Barrett-Joyner-Halenda (BJH) method.

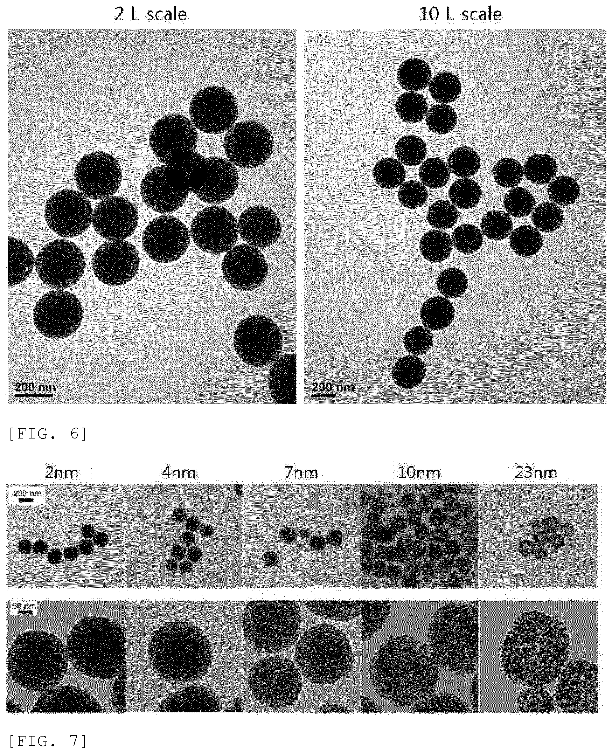

Micrographs of the particles are shown in FIG. 6, and the calculation results are shown in Table 1 below.

**[TABLE 1]**

| Section | Pore diameter (nm) | BET surface area (m²/g) |
|---|---|---|
| Small pore particles of Example 1-1-(1) | 2.1 | 1337 |
| Example 1-1-(7) | 4.3 | 630 |
| Example 1-1-(8) | 6.9 | 521 |
| Example 1-1-(1) | 17.2 | 377.5 |
| Example 1-1-(10) | 23 | 395 |
| Example 1-1-(11) | 12.3 | 379 |

### 4. Surface modification

100 mg of nanoparticles (BALL) of Example 1-1-(1) were dispersed in 10 mL toluene, and heated to 110 °C. When the temperature reached 110 °C, 2 ml of 3-aminopropyltriethoxysilane (APTES) was added and refluxed for 16 hours. Centrifugation was performed at 8500 rpm for 15 minutes to obtain particles (aBALL, in which a nitrogen-containing group is bonded to BALL), and washed twice with ethanol and distilled water alternately.

A precipitate was obtained and reacted with 0.1 mg of folic acid, 1.2 mg of N-hydroxysuccinimide (NHS) and 2 mg of N-(3-dimethylaminopropyl)-N'-ethylcarbodiimide hydrochloride (EDC) in ethanol.

Centrifugation was performed at 8500 rpm for 15 minutes to obtain particles (FaBALL, in which a nitrogen-containing group is bonded to BALL), followed by washing the same 10 times alternately with ethanol and distilled water.

The obtained particles were moved to HCl/hydrochloric acid solution (1:5 v/v) and refluxed at 110 °C for 16 hours. After centrifugation at 8500 rpm for 15 minutes, particles free of CTAB were obtained, followed by washing the same 10 times alternately with ethanol and distilled water.

The obtained particles were dispersed in 10 mL of toluene and heated to 110 °C. When the temperature reached 110 °C, 1 ml of 3-mercaptopropyltriethoxysilane (MPTES) was added and refluxed for 16 hours. After centrifugation at 8500 rpm for 15 minutes, particles (FabBALL, in which a mercaptopropyl group is bonded to FaBALL, and a sulfur atom ratio is 0.3 mmol/g in the pores) were obtained, and washed 10 times alternately with ethanol and distilled water.

### 5. Evaluation of particle properties

The obtained FabBALL was observed by TEM, and a particle diameter thereof was measured. Further, zeta potential of each BALL was measured (FIG. 7).

### 6. Evaluation of amount of folic acid

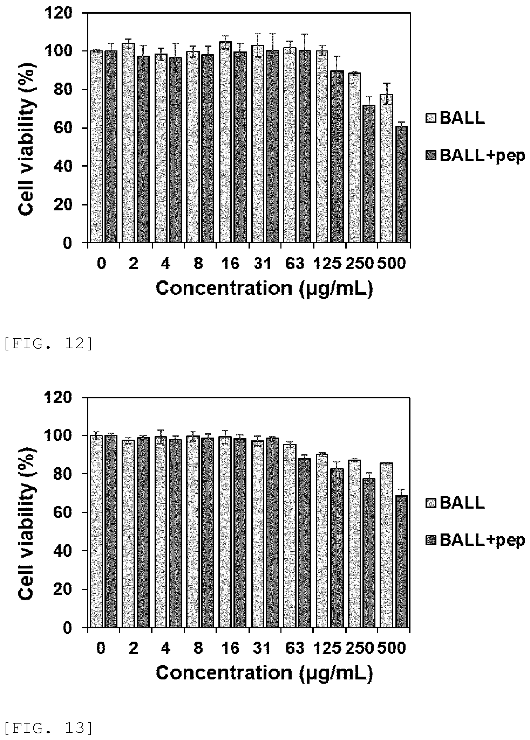

As shown in FIG. 13, surface modification was performed in the same manner as in Example 1-4, except that an amount of folic acid used for treatment was varied. Thereafter, whether or not particles are precipitated was confirmed.

The amount of folic acid relative to the nitrogen-containing groups (aminopropyl group) of the particles was set to 0%, 0.01%, 0.1%, 1%, 10% and 100%, which was adjusted by varying the added amount of folic acid. Specifically, the introduced amount of aminopropyl group was determined through elemental analysis (a nitrogen atom ratio of 2.1 mmol/g). Further, in order to match with a molar ratio of folic acid to the nitrogen-containing group of 1:1 (100%), 10:1 (10%), 100:1 (1%), 1000:1 (0.1%), and 10000:1 (0.01%), 19 mg, 1.9 mg, 0.19 mg, 0.019 mg and 0.0019 mg of folic acid, respectively, were added to 12 mg of the particles. Further, EDC was introduced in an amount of 41 mg, 4.1 mg, 0.41 mg, 0.041 mg and 0.0041 mg, respectively, while adding 25 mg, 2.5 mg, 0.25 mg, 0.025 mg, and 0.0025 mg of NHS, respectively.

The modified particles were added to PBS at a concentration of 8 mg/ml and, when the amount of folic acid was 1% or 10%, a precipitate was formed.

### 7. Dye labeling of particles

10 mg of the particles obtained in Example 1-2 were suspended in 1 ml of water, and 20 µg of N-hydroxysuccinimyl ester-activated Cyanine 3 (Cy3-NHS) was mixed thereto. The mixture was reacted for 16 hours under stirring. The unreacted fluorescent dye was removed by washing the particles 10 times with ethanol and water. Dye labeled particles were dried and redispersed in water.

### 8. Cell culture

MCF-7 cells (autophagy deficient breast cancer cell overexpressing folate receptor) were cultured in RPMI1640 medium mixed with 50 units/mL of penicillin/streptomycin solution and 10% fetal bovine serum (FBS).

### 9. Evaluation of intracellular influx

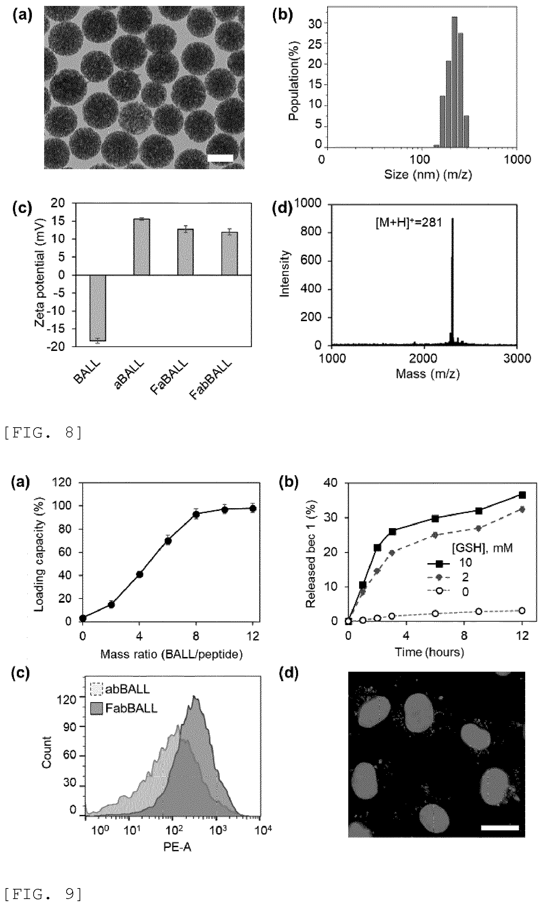

A difference in endocytosis efficiency into MCF-7 cells between FabBALL and abBALL was evaluated. Dye-labeled FabBALL and abBALL were treated with MCF-7 cells, and a fluorescence intensity was determined. The average fluorescence intensity was 2 times higher in FabBALL than abBALL (FIG. 8c).

### Example 2: Loading and release of peptide

### 1. Synthesis of peptides

Becl peptide (CGGTNVFNATFHIWHSGQFGT, SEQ ID NO: 1) was synthesized by solid phase synthesis using Rink amide resin. For fluorescent labeling, 3 eq fluorescein, 3 eq NHS and 3 eq EDC were added to the peptide. The obtained peptide was purified by HPLC and confirmed by MALDI-ToF mass spectrometry.

### 2. Measurement of loading efficiency

An excess of 4,4-dipyridyldisufide was added to the particles of Examples 1-2-5 and washed 3 times with ethanol. Then, different amounts of particles were added to a fixed amount of Becl peptide in a 20% DMSO solution and reacted for 1 hour. Centrifugation was conducted at 8500 rpm for 15 minutes to obtain a supernatant, and absorbance at 324 nm was measured. A concentration of the residual peptide was calculated by comparison with the standard curve of 4-thiopyridone. The peptide was loaded on FabBALL by about 10% w/w (FIG. 8a).

### 3. Peptide release

100 µg of the particles of Examples 1-2-4 and 10 µg of fluorescent-labeled Bec peptide were mixed in 160 µl of 1 x PBS with different concentrations of GSH. 40 µl of DMSO was added to dissolve the Becl peptide and the fluorescence intensity was measured. An amount of released peptide was calculated based on the initial fluorescence of the Becl peptide.

In the absence of GSH, only 3% or less of the peptide was released during 12 hours, whereas, in the presence of 10 mM or 2 mM glutathione (GSH), the peptide was released as much as 30% within 12 hours from FabBALL (FIG. 8b). This demonstrates that the release of the peptide is dependent on the reductive environment and is more promoted within the cytoplasm.

### 4. Assessment of cytotoxicity

MCF-7 cells were seeded at 10,000 cells/well in 96-well cell culture plates. After 1 day, different concentrations of each particle (4 to 500 µg/mL) were treated with a serum-containing medium in each well for 1 day. Thereafter, the culture was performed for 4 hours while replacing the above medium with a serum-free medium containing 10 µl of MTT assay kit, and the supernatant was aspirated. DMSO was added to each well to dissolve formazan, and reacted for 1 hour. The absorbance was measured at a wavelength of 570 nm, and the cell viability was calculated as a relative value to that of the untreated group.

Cell viability was compared by MTT assay after treatment with FabBALL and Becl peptide carrying Becl peptide on MCF-7 cells. When only the Becl peptide was used for treatment, the cell viability was not significantly varied compared to the non-treated group. However, when the FabBALL carrying the Becl peptide was used for treatment, the cell viability was significantly reduced (FIGS. 9a and 9b). It was confirmed that FabBALL exhibited high efficiency in target delivery of Becl peptide.

### 5. Fluorescent image of cells for determination of intracellular peptide release

MCF-7 cells were seeded at 100,000 cells/well in a 12-well cell culture plate with a glass bottom. After 1 day, 5 µg of dye-labeled particles and 0.5 µg of fluorescence-labeled bec1 peptide were mixed together, and cells were treated with complexes under a serum-free medium for different periods (1, 2, 3, 6, 12, 24 hours), respectively. Cells treated with the particles were fixed with 4% PFA solution for 15 minutes and then washed twice with 1 x PBS solution. Cell nuclei were stained with DAPI solution. Images were acquired with a Delta-vision microscope.

It was confirmed that the Becl peptide was effectively released by processing the fluorescence-labeled FabBALL to MCF-7 cells. After 12 hours, the fluorescence of the green fluorescence-labeled Becl peptide was confirmed in a significant amount, showing that the peptide was well released in the cytoplasm from FabBALL (FIG. 8d).

### 6. Construction of MCF-7 cell line expressing LC3 GFP and measurement of the number of GFP-LC3 puncta

Using lipofectamine 2000, LC3-GFP plasmid was transfected into MCF-7 cells. Transfected MCF-7 was selected with 800 µg/mL of G418 for 3 months. GFP expression was observed for at least 1 month to confirm that the plasmid was properly inserted. 100,000 MCF-7 cells expressing LC3-GFP were seeded.

40 µg of dye-labeled particles and 4 µg of bec1 peptide were mixed, and the cells were treated for 6 hours in a serum-free medium. Cells were fixed with 4% PFA, and nuclei were stained with DAPI. Images were obtained with a Delta-vision microscope, and the number of GFP-LC3 puncta was measured in 100 cells for each group.

LC3 protein is a biomarker involved in the elongation process of autophagosome, and its expression level is known to increase with the progress of autophagosome. In this respect, an increase in GFP puncta shows that autophagy was successfully induced. When only Becl peptide was treated, a very little amount of GFP puncta was observed. However, when the FabBALL carrying the Becl peptide was treated, a large amount of GFP puncta was confirmed (FIGS. 9c and 9d).

### 7. Confirmation of effects in various cancer cell lines

Cytotoxicity in PC-3, LNCaP, and HeLa cells, respectively, was assessed in the same manner as in the assessment of cytotoxicity in MCF-7.

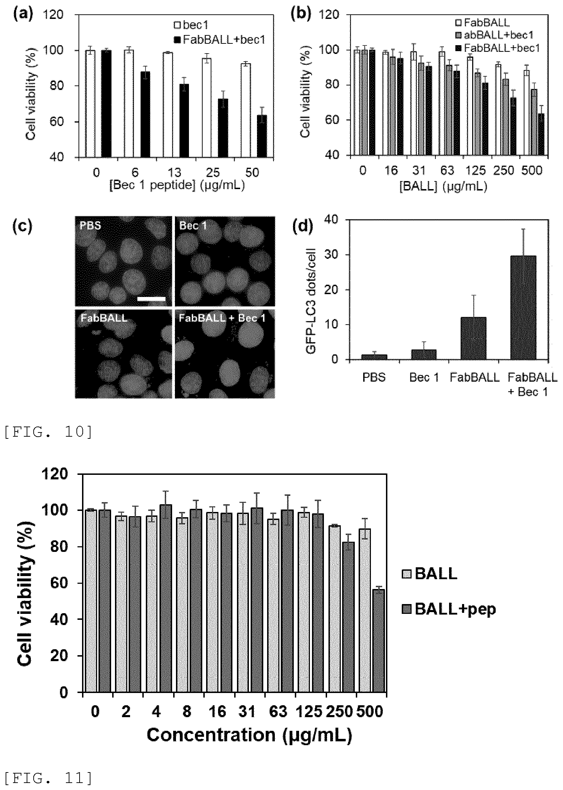

In all of these cell lines, it was confirmed that effective effects were exhibited when FabBALL carrying Becl peptide was used for treatment of the cell line (FIGS. 10 to 12) .

## Claims

1. An anticancer drug comprising a plurality of porous silica particles having an anticancer active peptide incorporated therein, wherein a plurality of nitrogen-containing groups are located on an outer surface of each porous silica particle, and folic acid is bound to at least some of the nitrogen-containing groups.

2. The anticancer drug according to claim 1, wherein the folic acid is bound to 0.9% or less of the nitrogen-containing groups.

3. The anticancer drug according to claim 1, wherein the folic acid is bound to 11% or more of the nitrogen-containing groups.

4. The anticancer drug according to claim 1, wherein the folic acid is bound to 0.001% to 0.3% of the nitrogen-containing groups.

5. The anticancer drug according to claim 1, wherein the anticancer active peptide is bound to the porous silica particles through a disulfide bond.

6. The anticancer drug according to claim 1, wherein the porous silica particles include a plurality of irregularly arranged pores.

7. The anticancer drug according to claim 1, wherein the porous silica particles have a particle diameter of 50 to 500 nm and a pore diameter of 7 to 25 nm.

8. The anticancer drug according to claim 1, wherein the anticancer active peptide has a length of 5aa to 50aa in length.

9. The anticancer drug according to claim 1, wherein the anticancer active peptide includes a linker having an amino acid sequence of C(GG)n (n is 1 to 3) at a terminal thereof.

10. The anticancer drug according to claim 1, wherein the anticancer active peptide has an amino acid sequence of SEQ ID NO: 1.

11. The anticancer drug according to claim 1, wherein the porous silica particles have a BET surface area of 280 m²/g to 680 m²/g.

12. The anticancer drug according to claim 1, wherein the anticancer active peptide is incorporated in the porous silica particles in a weight ratio of 1:1 to 20.

13. The anticancer drug according to claim 1, wherein the anticancer drug is an injectable formulation.

14. The anticancer drug according to claim 1, wherein the cancer is breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, bone cancer, hair cell cancer, oral cancer, lip cancer, tongue cancer, salivary gland cancer, pharyngeal cancer, small intestine cancer, colon cancer, rectal cancer, kidney cancer, prostate cancer, vulvar cancer, thyroid cancer, bowel cancer, endometrial cancer, uterine cancer, brain cancer, central nervous system cancer, peritoneal cancer, hepatocellular carcinoma, Hodgkin disease or leukemia.

15. The anticancer drug according to claim 1, wherein an amount of nitrogen atoms on the outer surface of the porous silica particles is 0.1 mmol/g or more.

16. A method for preparation of porous silica particles comprising an anticancer active peptide incorporated therein, the method comprising:
a first step of introducing nitrogen-containing groups on an outer surface of the porous silica particles in a state in which inner pores of the porous silica particles are filled with a surfactant;
a second step of binding folic acid to at least some of the nitrogen-containing groups;
a third step of removing the surfactant filled in the inner pores of the porous silica particles; and
a fourth step of incorporating active peptide in the porous silica particles.

17. The method according to claim 16, wherein the surfactant is selected from the group consisting of cetyltrimethylammonium bromide (CTAB), hexadecyltrimethylammonium bromide (TMABr), hexadecyltrimethylpyridinium chloride (TMPrCl), and tetramethylammonium chloride (TMACl).

18. The method according to claim 16, wherein the active peptide is bound to the porous silica particles in a weight ratio of 1:1 to 20.

19. The method according to claim 16, wherein the porous silica particles are treated with the folic acid in an amount of 0.01 to 10 parts by weight based on 100 parts by weight of the porous silica particles.

20. The method according to claim 16, further comprising a step of preparing small pore silica particles in which small pores are filled with the surfactant by adding the surfactant and a silica precursor in a solvent and then stirring the same prior to the first step.

21. The method according to claim 20, further comprising a step of expanding the small pores by reacting the small pore silica particles with a swelling agent prior to the first step.

22. A method for production of the anticancer drug according to any one of claims 1 to 15, comprising the method for preparation of porous silica particles according to any one of claims 16 to 21.


<110>    LEMONEX INC.

<120>    ANTICANCER DRUG AND PREPARING METHOD FOR POROUS SILICA PARTICLE

<130>    20P07004

<150>    US 62/880733
<151>    2019-07-31

<160>    1

<170>    KoPatentIn 3.0

<210>    1
<211>    21
<212>    PRT
<213>    Homo sapiens

<400>    1
Cys Gly Gly Thr Asn Val Phe Asn Ala Thr Phe His Ile Trp His Ser
  1               5                  10                  15

Gly Gln Phe Gly Thr
             20